# EUROPEAN PATENT APPLICATION

(11) **EP 2 418 215 A1**
(43) Date of publication of application: **15.02.2012**
(21) Application number: 10761197.2
(22) Date of filing: 07.04.2010
(51) Int. Cl.: C07K 14/415, C12N 15/29, C12N 15/63, C12N 15/11, C12Q 1/68, A01H 1/00

(54) **RICE ZINC FINGER PROTEIN TRANSCRIPTION FACTOR DST AND USE THEREOF FOR REGULATING DROUGHT AND SALT TOLERANCE**

(30) Priority: 08.04.2009 CN 200910048955
(71) Applicant: Shanghai Institutes for Biological Sciences, CAS, Shanghai 200031 (CN)
(72) Inventor: LIN, Hongxuan, Shanghai 200031 (CN); HUANG, Xinyuan, Shanghai 200031 (CN); CHAO, Daiyin, Shanghai 200031 (CN); GAO, Jiping, Shanghai 200031 (CN); ZHU, Meizhen, Shanghai 200031 (CN); SHI, Min, Shanghai 200031 (CN)
(74) Representative: Giovannini, Francesca
(86) International application number: PCT/CN2010/071587
(87) International publication number: WO 2010/115368

(57) **Abstract**

Provided are zinc finger protein transcription factor DST having the amino acid sequence as shown in SEQ ID NO: 2, conservative variants and homologous polypeptides thereof. Also provided are DNA sequence encoding the transcription factor DST, vector or host cell comprising the DNA sequence, cis-acting element binding to the DST, inhibitor or non-conservative variant of the transcription factor DST or encoding sequence thereof, and use of the inhibitor or non-conservative variant for improving the drought and salt tolerance in plant.

## Description

### TECHNICAL FIELD

The present invention relates to the fields of plant bioengineering and genetic engineering for plant improvement. Specifically, the present invention relates to use of novel rice zinc finger protein transcription factor genes and their encoded proteins or polypeptides to increase drought and salt tolerance in plants, methods for improving salt resistance and/or drought resistance in plants by inhibiting genes described above or their expressed proteins, and transgenic plants.

### BACKGROUND ART

Increased global demands for food and continued shrinkage of farmland have created constant pressure on national food security. In food production, drought and salts are major non-biological (abiotic) stresses, causing significant reduction in quantities and qualities of crops every year. In addition, drought often accompanies salts. For example, salinization of soil is a common phenomenon of soil deterioration in drought areas.

Available data show that annual rice losses due to drought in China cost at least $2 billions, and salinization of farmland is also the main reason for reduced productions and low yields. Drought and salts have represented two serious problems confronting China's agriculture.

Therefore, how to improve drought and/or salt tolerance in crops to increase yields, solving China's and world's food problems, is of great significance. The abiotic stress research is one of the most urgent and challenging fields in plant research.

To date, some drought and salt resistance related genes, including several transcription factors, have been cloned, and some stress-resistant strains of plants or crops have been obtained by using genetic engineering techniques. The aim of stress-resistance genetic engineering is to improve plant stress resistance by modulating gene transcription and expression, in which transcription factors play key roles.

At present, some transcription factors that participate in stress-response gene expression have been cloned. However, due to so many transcription factors participating in these complex processes of stress response in plants, these transcription factors only represent the tip of an iceberg. Much more transcription factors remain to be discovered, investigated, and used in breeding crops with stress resistance.

Therefore, there is an urgent need in this field to investigate transcription factors that participate in stress-response gene expression, to develop new methods for breeding stress-resistant crops and to develop such new crops, thereby improving quantities and qualities of crops.

### SUMMARY OF INVENTION

One objective of the present invention is to provide a novel gene that is closely related to salt and drought tolerance in plants (especially crops) -- rice zinc finger protein transcription factor DST, and to confirm that this transcription factor is a negative regulator of salt and drought tolerance in plants. Another objective of the present invention is to provide a novel way to enhance salt and/or drought stress resistance in plants. Another objective of the present invention is to provide methods for generating transgenic plants with enhanced drought and salt tolerance and transgenic plants generated by these methods. Another objective of the present invention is to provide methods for screening for plants having enhanced drought and salt resistance and plants obtained by these screening methods.

In the first aspect, the present invention provides isolated zinc finger protein transcription factors, including polypeptides having the amino acid sequence of SEQ ID NO: 2, polypeptides having conserved mutations in the preceding polypeptides, or homologs of the preceding polypeptides.

In one preferred embodiment, the above polypeptides includes a Cys-2/His-2 type zinc finger structural domain.

In another preferred embodiment, said polypeptides participates in the regulation of genes related to enzymes that work on peroxides, controlling hydrogen peroxide accumulation and/or regulating stomatal aperture, thereby affecting drought and salt tolerance in rice.

In one embodiment of the present invention, said polypeptide is selected from the following group:
(a) a polypeptide having the amino acid sequence of SEQ ID NO: 2;
(b) a polypeptide derived from (a) with one or more amino acid residue substitutions, deletions, or insertions, and capable of increasing drought and salt sensitivity in a plant; or
(c) a polypeptide homolog of the polypeptide of (a) or (b) having a Cys-2/His-2 type zinc finger structural domain and capable of increasing drought and salt sensitivity in a plant.

In a preferred embodiment, said plants are dicotyledonous plants or monocotyledonous plants, preferably crops.

In another preferred embodiment, said plants are selected from: Gramineae, Malvaceae gossypium, Cruciferae brassica, Compositae, Solanaceae, Labiatae, or Umbelliferae, preferably Gramineae.

In another preferred embodiment, said plants are selected from: rice, corn, wheat, barley, sugar cane, sorghum, Arabidopsis, cotton or canola, more preferably rice, corn, wheat, barley, sugar cane or sorghum.

In another preferred embodiment, said salts refer to: sodium chloride, sodium sulfate, sodium carbonate or sodium bicarbonate.

In the second aspect, the present invention provides isolated polynucleotides, which include nucleotide sequences coding for polypeptides of present invention.

In one preferred embodiment, said polynucleotide encodes the amino acid sequence of SEQ ID NO: 2 or its polypeptide homolog.

In one embodiment of the present invention, said polynucleotide sequence is one selected from the following:
(a) a nucleotide sequence comprising the sequence of SEQ ID NO: 1;
(b) a nucleotide sequence comprising nucleotides 1-435 in SEQ ID NO: 1; or
(c) a polynucleotide sequence complementary to one of the nucleotide sequences of (a)-(b).

In the third aspect, the present invention provides a vector, which contains a polynucleotide of the present invention.

In a preferred embodiment, said vector is selected from: a bacterial plasmid, a phage, a yeast plasmid, a plant virus, or a mammalian virus; preferably, pCAMBIA1301, pEGFP-1, pBI121, pCAMBIA1300, pCAMBIA2301 or pHB, and, more preferably, pCAMBIA1301.

In the fourth aspect, the present invention provides genetically engineered host cells, which contain a vector of the present invention or having a polynucleotide of the present invention integrated into the genome.

In a preferred embodiment, said host cell is selected from a prokaryotic cell, a lower eukaryotic cell or a higher eukaryotic cell, preferably a bacterial cell, a yeast cell or a plant cell, more preferably E. coli, Streptomyces, Agrobacterium, yeast, most preferably Agrobacterium, said Agrobacterium includes, but is not limited to: EHA105, SOUP1301 or C58, preferably, EHA105.

In the fifth aspect, the present invention provides a cis-acting element, which includes the sequence of SEQ ID NO: 3, capable of binding to a transcription factor of the present invention.

In a preferred embodiment, said cis-acting element has the sequence of TGCTANN(A/T)TTG, in which N is selected from A, C, G or T.

In another preferred embodiment, said cis-acting element binds to a zinc finger structural domain of a transcription factor of the present invention.

In another preferred embodiment, binding of said cis-acting element to a transcription factor of the present invention can increase the sensitivity to drought and salt in plants.

In the sixth aspect, the present invention provides antagonists for zinc finger transcription factor proteins or polynucleotides.

In a preferred embodiment, antagonists are small interference RNAs, antibodies or antisense oligonucleotides.

In the seventh aspect, the present invention provides methods to improve drought and salt tolerance in plants, said methods include inhibiting zinc finger transcription factors of the present invention, inhibiting the expression of polynucleotides of the present invention, or inhibiting the binding of cis-acting element to zinc finger protein transcription factors of the present invention.

In one preferred embodiment, said inhibition is carried out by methods of deletion, mutation, RNAi, antisense or dominant negative regulation.

In another preferred embodiment, said inhibition includes introducing one or more amino acids or nucleotide substitution, deletion, or insertion to transcription factors of the present invention or polynucleotides of the present invention, resulting in said plants having improved drought and salt tolerance.

In another preferred embodiment, according to the amino acid sequence of SEQ ID NO: 2, said inhibition involves mutating asparagine to aspartic acid at amino acid 69, mutating alanine to threonine at amino acid 162, resulting in plants with these mutant sequences to have improved drought and salt tolerance.

In another preferred embodiment, said methods include applying an antagonist of the present invention to a plant.

In another preferred embodiment, said inhibition includes: transforming plants with a vector containing a small interference RNA targeting a zinc finger protein transcription factor of the present invention, or transforming plants using a host cell containing said vectors.

In another preferred embodiment, said methods further include cross-breeding plants having enhanced drought and salt tolerance obtained by the methods described above with non-transgenic plants or other transgenic plants.

In another preferred embodiment, said salts refer to: sodium chloride, sodium sulfate, sodium carbonate or sodium bicarbonate.

In the eighth aspect, the present invention provides methods for screening for plants with drought and salt tolerance, said methods include:
(i) detecting in a candidate plant the level of a zinc finger protein transcription factor of the present invention, the expression level of a polynucleotide of the present invention, and/or the binding levels of a cis-acting element of the present invention to a zinc finger protein transcription factor of the present invention; and
(ii) comparing the level in the candidate plant detected in step (i) with the level in a control plant, if the level in the candidate plant is lower than that of the control plant, said candidate plant is a drought and salt tolerant plant.

In a preferred embodiment, said salts refer to: sodium chloride, sodium sulfate, sodium carbonate or sodium bicarbonate.

In the ninth aspect, the present invention provides methods for preparing a zinc finger protein transcription factor, **characterized in that,** said methods include:
(a) culturing a host cell of the present invention under conditions suitable for expression; and
(b) isolating a zinc finger protein transcription factor from the culture media.

In another aspect, the present invention provides uses of an inhibitor or a non-conserved mutant sequence of a zinc finger protein transcription factor or a nucleotide sequence of the present invention to improve drought and salt tolerance in a plant.

In one preferred embodiment, said inhibitor is a small interference RNA, an antibody, or an antisense oligonucleotide, which targets said transcription factor or nucleotide sequence.

In another preferred embodiment, said non-conserved mutant sequence inhibits translation or expression of a zinc finger protein transcription factor or a nucleotide sequence of the present invention in a plant containing said non-conserved mutant sequence, resulting in better drought and salt tolerance than that of a wild-type plant, that does not contain the non-conserved mutant sequence.

In another preferred embodiment, said non-conserved mutant sequence is the polynucleotide sequence of SEQ ID NO: 1 with two mutations: A at position 205 is mutated to G and G at position 484 is mutated to A; or the amino acid sequence of SEQ ID NO: 2 with two mutations: asparagine at position 69 is mutated to aspartic acid and alanine at position 162 is mutated to threonine.

In one embodiment of the present invention, said improving drought and salt tolerance in plants includes:
(i) directly applying an inhibitor (antagonist) described above to a plant;
(ii) introducing a non-conserved mutant sequence described above into a plant; or
(iii) designing a molecular marker specific for the non-conserved mutant sequence, and using said molecular marker to screen offsprings derived from cross-breeding a mutant plant having the non-conserved mutant sequence with another rice species to select for an individual offspring containing the non-conserved mutant sequence.

In one preferred embodiment of the present invention, said molecular marker comprises a primer pair having the sequences shown in SEQ ID NO: 10 and SEQ ID NO: 11, and/or a primer pair having the sequences shown in SEQ ID NO: 12 and SEQ ID NO: 13.

In another aspect, the present invention provides methods for improving drought and salt tolerance in a plant, said method include: (A) providing an inhibitor or a non-conserved mutant sequence for a zinc finger protein transcription factor or a polynucleotide sequence of the present invention; (B) subjecting a plant to one or more treatments selected from the following: (i) applying said inhibitor directly to the plant; (ii) introducing the non-conserved mutant sequence into the plant; or (iii) designing a molecular marker specific for the non-conserved mutant sequence, and using said molecular marker to screen offsprings from cross-breeding of a mutant plant having the non-conserved mutant sequence and another rice strain to select for an individual offspring that contains the non-conserved mutant sequence.

In one preferred embodiment of the present invention, said molecular marker is a primer pair having the sequences shown in SEQ ID NO: 10 and SEQ ID NO: 11, and/or a primer pair having the sequences shown in SEQ ID NO: 12 and SEQ ID NO: 13.

In another aspect, the present invention provides methods for preparing a transgenic plant, said methods include:
(1) transfecting a plant cell, a plant tissue or a plant organ with a construct containing a non-conserved mutant sequence of a zinc finger protein transcription factor of the present invention or a construct containing a non-conserved mutant sequence of a polynucleotide of the present invention;
(2) selecting for a plant cell, a plant tissue, or a plant organ containing the non-conserved mutant sequence; and
(3) regenerating a plant from the plant cell, the plant tissue or the plant organ obtained in step (2),
wherein the obtained transgenic plant has higher drought and salt tolerance than a non-transgenic plant.

In another preferred embodiment, said methods also include cross-breeding the obtained transgenic plant with a non-transgenic plant or another transgenic plant, thereby obtaining a hybrid offspring containing the non-conserved mutant sequence, said hybrid offspring has higher drought and salt tolerance than a non-transgenic plant, preferably said hybrid offspring has stable genetic traits.

In another preferred embodiment, said methods also include designing a molecular marker specific for the non-conserved mutant sequence to screen offsprings obtained from cross-breeding of the transgenic plant to obtain a plant having improved drought and salt tolerance.

Based on the present description, other aspects of the invention whould be apparent to one skilled in the art.

### DESCRIPTION OF DRAWINGS

FIG. 1: Rice DST gene sequence (FIG. 1A) and its encoded amino acid sequence (FIG. 1B).
FIG. 2: Comparison of the phenotypes of rice DST gene mutant dst and the phenotypes of wild type rice under drought and salt conditions. In each panel, the wild type (Zhonghua 11, ZH11) is on the left and the dst mutant is on the right.
FIG. 3: Comparison of phenotypes, under drought and salt conditions, of the wild type, dst mutant obtained by DST gene complementation, and plants with reduced DST function by RNAi.
FIG. 4: Analysis of DST transcriptional activation using MatchmakerTM GAL4 yeast two-hybrid system 3 (Clontech).
FIG. 5: Results electrophoresis mobility shift assay (EMSA).
FIG. 6: Sequence alignment analysis of homologous DST zinc finger protein domains among Gramineae crops.

### DETAILED DESCRIPTION

After a long and intensive investigation, inventors of the present invention discovered a novel rice zinc finger protein transcription factor gene DST (Drought and Salt Tolerance gene) and confirmed that this gene is a negative regulatory factor for drought and salt tolerance, capable of controlling drought and salt tolerance in plants, and that inhibiting the expression of this gene can increase resistance to salt or drought stress in plants. Thus, this gene plays an important role in breeding plants that are resistant to drought and salt. Based on these, the inventors have reduced the invention to practice.

Specifically, using a rice mutant library (EMS mutagenesis) and map-based cloning techniques, the inventors performed a large-scale screening under salt stress conditions to obtain a novel gene DST that controls drought and salt tolerance in rice. The length of genomic DST gene is 906bp, which does not include any intron. Therefore, the full-length ORF (open reading-frame) is 906bp. This gene encodes 301 amino acids, a protein of about 29KDa that includes a conserved zinc finger domain. This protein is a transcription factor.

Results from phenotype identification show that mutants of this gene (for example, DST gene with 2 nucleotide mutations, resulting in 2 amino-acid substitutions) exhibit both drought and salt tolerance. Using RNAi to down regulate the expression of this gene also produced enhanced drought and salt tolerance.

Results from biochemical studies show that DST is a transcription factor that includes not only a transcription activation domain, but also a DNA binding domain. Gene chip analysis shows that DST functions as a transcription factor that regulates a series of downstream genes.

Functional studies show that, as compared with the wild-type, mutants have more hydrogen peroxide (H2O2) accumulated around stomata, have smaller stomatal apertures, and allow leaves to maintain relatively higher water contents under drought stress. Therefore, mutants have higher drought tolerance. In addition, due to smaller stomatal apertures in mutants, stomatal conductance is lower, and the rate of water vaporization is slower. As a result, transportation of Na+ ions from roots to parts above ground (leaves, etc.) is reduced, and therefore Na+ toxicity is lower, thereby enhancing salt tolerance. This study shows that DST participates in the regulation of peroxidase-related genes, controls hydrogen peroxide (H2O2) accumulation, regulates stomatal aperture, thereby affecting drought and salt tolerance in rice.

The above studies show that DST gene is a negative regulatory factor for drought resistance and salt resistance, inhibiting its expression can enhance resistance to salt or drought stress in plants. This property can be used to produce transgenic plants with significantly higher resistance to salt stress and drought. Thus, DST gene has a great potential in improving the ability of crops to tolerate adverse stresses, such as salt stress and drought.

Furthermore, database search reveals: one DST homologous gene in sorghum (Sorghum bicolor) genome, with protein similarity of 54.3%; three DST homologous genes in maize (Zea mays) genome, with protein similarities of 51.7%, 36.1%, and 33.5%; one DST homologous gene in barley (Hordeum vulgare) genome, with protein similarity of 38.4%; and three DST homologous genes in sugar cane (Saccharum officinarum) genome, with protein similarities of 38.2%, 38.2% and 34.5%. These homologous genes all have a conserved C2H2-type zinc finger structural domain, with high similarities at the N terminus, suggesting that DST homologous genes in other plants (preferrably, Gramineae) would have similar functions as that of rice DST gene.

### DST PROTEINS OR POLYPEPTIDES AND THEIR CODING SEQUENCES

In the present invention, the terms "DST proteins or polypeptides," "DST gene encoded proteins or polypeptides," or "zinc finger protein transcription factors" refer to proteins or polypeptides encoded by DST genes of the present invention. These definitions include mutants of the above-described proteins or polypeptides with conserved mutations, or their homologous polypeptides. They all have Cys-2/His-2 type zinc finger structural domains, and, when the expression of said proteins or polypeptides is inhibited, the resistance to drought or salt stresses can be increased in plants.

In one embodiment of the present invention, said transcription factors participate in regulation of peroxidase-related genes, control hydrogen peroxide accumulation and/or regulate stomatal aperture, thereby affecting drought and salt tolerance in rice.

Said DST protein or polypeptide sequences are selected from: (a) polypeptides having the amino acid sequence of SEQ ID NO: 2; (b) polypeptides derived from (a) having one or more amino acid residue substitutions, deletions or insertions in the amino acid sequence of SEQ ID NO: 2, and capable of increasing drought and salt susceptibility in plants; or (c) polypeptide homologs of the polypeptides of (a) or (b) having Cys-2/His-2 type zinc finger structural domains and capable of increasing drought and salt susceptibility in plants. Preferably, said proteins or polypeptides can bind to TGCTANN(A/T)TTG, wherein N represents A, C, G or T.

Proteins and polypeptides of the present invention can be purified natural products, or chemically synthesized products, or produced, by using recombinant technology, from prokaryotic or eukaryotic host cells (for example, bacteria, yeast, higher plants, insects and mammalian cells). DST proteins or polypeptides of the present invention, preferably are encoded by Gramineae (preferably, rice) DST gene or its homologous genes or family genes.

Types of mutations in proteins or polypeptides of the present invention include, but are not limited to: deletion, insertion and/or substitution of one or more (usually 1-50, preferably 1-30, more preferably 1-20, most preferably 1-10, for example, 1, 2, 3, 4, 5, 6, 7, 8, 9, or 10) amino acid, and addition at the C terminus and/or N terminus of one or several (usually 20 or less, preferably fewer than 10, most preferably fewer than 5) amino acids. For example, it is known in the art that, when substituting amino acids having related or similar properties, the functions of proteins or polypeptides usually do not change. As another example, addition of one or several amino acids at the C terminus and/or N terminus usually does not change the functions of proteins or polypeptides. For example, DST proteins or polypeptides of the present invention may or may not include the starting methionine residue and still have the activity to increase resistance to heavy metals or salt stress in plants. One skilled in the art, based on common knowledge in the art and/or routine experimentation, can easily identify these various types of mutation that would not affect the activity of proteins and polypeptides.

In the present invention, the term "conserved mutant polypeptides" refers to polypeptides, as compared with the amino acid sequence of SEQ ID NO: 2, having up to 20, preferably up to 10, more preferably up to 5, most preferably up to 3 amino acids substituted with amino acids having related or similar properties. These conserved mutant polypeptides can be best generated according to the following table for amino acid substitutions:

| Amino Acid Residues | Representative Substitutions | Preferred Substitutions |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

Said proteins and polypeptides from (b) can be obtained by exposure to radiation or mutagens to produce random mutagenesis, or through site-directed mutagenesis or other known molecular biology techniques. The sequences encoding the proteins or polypeptides can be used to construct transgenic plants to screen for and identify the proteins or polypeptides based on whether the transgenic plants have altered characteristics.

Mutant forms of said polypeptides include: homologous sequences, conserved mutants, allelic mutants, natural mutants, induced mutants, proteins encoded by sequences that can hybridize with the coding sequences for DST protein under high or low stringent conditions, and polypeptides or proteins obtained using anti-DST protein antiserum. Other polypeptides can also be used in the present invention, such as fusion proteins containing a DST protein or its fragment. In addition to the almost full-length polypeptides, the present invention also includes soluble fragments of the DST proteins. Generally, said soluble fragments contain at least about 10 consecutive amino acids in the DST protein sequence, usually at least about 30 consecutive amino acids, preferably at least about 50 consecutive amino acids, more preferably at least about 80 consecutive amino acids, most preferably at least about 100 consecutive amino acids.

Depending on hosts used for producing recombinants, proteins or polypeptides of the present invention may be glycosylated, or may be non-glycosylated. The term also includes active fragments and active derivatives of the DST proteins.

In the present description, the terms "DST gene," "plant DST gene," or "coding sequences of transcription factors of the present invention" are interchangeable. They all refer to sequences coding for the DST proteins or polypeptides of the present invention. They are highly homologous to rice DST gene sequence (see SEQ ID NO: 1); they are molecules that can hybridize with said gene sequence under stringent conditions; or they are family gene molecules highly homologous to said molecules. Inhibiting said gene expression results in definite improvement in the resistance to drought or salt stress in plants.

In one embodiment of the present invention, said polynucleotide includes: (a) the nucleotide sequences of SEQ ID NO: 1; (b) a nucleotide sequence having nucleotides 1-435 of SEQ ID NO: 1; or (c) polynucleotides complementary to one of the nucleotide sequences in (a)-(b).

In the present description, the term "stringent conditions" refers to: (1) hybridization and washing under low ionic strength and high temperatures, such as 0.2× SSC, 0.1% SDS, 60°C; or (2) hybridization in the present of a denaturing agent, such as 50% (v/v) formamide, 0.1% calf serum/0.1% Ficoll, 42°C, etc.; or (3) hybridization that occurs only when the homology between the two sequences reaches at least50%, preferably 55% or more, 60% or more, 65% or more, 70% or more, 75% or more, 80% or more, 85% or more, or 90% or more, more preferably 95% or more. For example, said sequences can be sequences complementary to the sequences defined in (a).

Full-length or fragments of DST gene nucleotide sequences of the present invention can usually be obtained by PCR amplification, recombination or synthetic methods. For PCR amplification, related sequences can be obtained by designing primers based on related nucleotide sequences disclosed in the present invention, specifically the open reading frame, and using commercially available cDNA libraries or cDNA libraries generated with common methods known by a skilled artisan in the art as templates. When dealing with long sequences, two or more PCR amplification are usually needed, and then assemble the fragments obtained from amplification according to the correct orders.

It should be understood that DST gene of the present invention is preferably from rice. Other genes obtained from other plants that share high homology with rice DST gene (such as 50% or more, preferably 55% or more, 60% or more, 65% or more, 70% or more, 75% or more, 80% or more, more preferably 85% or more, such as 85%, 90%, 95% or even 98% of sequence identity) are also considered to be within the scope of the present invention. Methods and tools for comparing sequence identity are also well known in the art, such as BLAST.

### PLANTS AND THEIR RESISTANCE TO SALT AND/OR DROUGHT STRESS

As used in the present description, said "plants" include (but is not limited to): Gramineae, Malvaceae Gossypium plants, cruciferous Brassica, Compositae, Solanaceae, Labiatae plants or Umbelliferae, etc. Preferably, said plants are Gramineae plants, more preferably Gramineae crops. For example, said plants may be selected from: rice, corn, wheat, barley, sugar cane, sorghum, Arabidopsis, cotton or canola, more preferably rice, corn, wheat, barley, sugar cane or sorghum.

As used in the present description, the term "crops" refers to plants of economic values in the grain, cotton, oil, etc. agriculture and industry. The economic values can be reflected by plants' seeds, fruits, roots, stems, leaves and other useful parts. Crops include, but are not limited to: dicotyledons or monocotyledons. Preferred monocotyledonous plants are Gramineae plants, more preferably rice, wheat, barley, corn, sorghum and so on. Preferred dicotyledons include, but are not limited to: Malvaceae cotton plants, cruciferous plants such as Brassica, more preferably cotton and canola.

As used in the present description, the term "salt stress" refers to: a phenomenon that, when plants grow in soil or water containing high concentration of salts, their growths would be inhibited or even they would die. Salts that cause salt stress include (but are not limited to): sodium chloride, sodium sulfate, sodium carbonate or sodium bicarbonate. DST genes of the present invention or their encoded proteins or polypeptides can increase the resistance to salt stress in plants. The increased resistance can be observed by comparison with control plants that have not been treated with said genes, proteins, or polypeptides. The growth and development of said plants are not affected or less affected by high salt concentrations, or said plants can survive in higher salt concentrations.

As used in the present description, the term "drought stress" refers to: a phenomenon that when plants grow in dry soil or other drought environments, their growths would be inhibited or even they would die. DST genes of the present invention or their encoded proteins or polypeptides can increase the resistance of plants to drought stress, the increased resistance can be observed by comparison with control plants that have not been treated with said genes, proteins, or polypeptides. The growth and development of said plants are not affected or less affected by lack of water, or said plants can survive in hasher drought conditions.

### VECTORS, HOSTS, AND TRANSGENIC PLANTS

The present invention also relates to vectors containing DST genes, and host cells containing said vectors generated by genetic engineering, and transgenic plants generated by gene transfection and expressing high levels of DST.

Using conventional recombinant DNA technology (Science, 1984; 224:1431), coding sequences of the present invention can be used to express or produce recombinant DST proteins. In general, these involve the following steps:
(1) transfecting or transforming suitable host cells with polynucleotides (or mutants) encoding DST proteins of the present invention, or with recombinant expression vectors containing said polynucleotides;
(2) culturing the host cells in suitable culture media; and
(3) isolating and purificating proteins or polypeptides from the culture media or the cells.

In the present invention, the terms "vectors" and "recombinant expression vectors" can be used interchangeably, referring to, bacterial plasmids, bacteriophages, yeast plasmids, plant cell viruses, mammalian cell viruses or other vectors, which are well known in the art. In short, any plasmids and vectors can be used as long as they can replicate and are stable inside host cells. One important feature of expression vectors is that they usually contain a replication origin, a promoter, a marker gene and a translational control element.

Methods well known to one skilled in the art can be used to construct expression vectors containing a DST coding sequences and a suitable transcriptional/translational control signal. These methods include in vitro recombinant DNA technology, DNA synthesis technology, and in vivo recombination technology, etc. Said DNA sequences can be effectively linked to suitable promoters in expression vectors, directing mRNA synthesis. Expression vectors also include ribosome binding sites for translation initiation and transcription termination sites. In the present invention, pEGFP-1, pBI121, pCAMBIA1300, pCAMBIA1301, pCAMBIA2301 or pHB is preferably used.

In addition, expression vectors preferably include one or more selection marker genes, providing phenotypes for the selection of transfected host cells, such as dihydrofolate reductase, neomycin resistance and green fluorescent protein (GFP) for use in eukaryotic cell culture, or tetracycline or ampicillin resistance for use in E. coli.

Vectors containing the above-described DNA sequences and suitable promoters or control elements can be used to transform suitable host cells, enabling them to express proteins or polypeptides. Host cells can be prokaryotic cells, such as bacterial cells; or lower eukaryotic cells, such as yeast cells; or higher eukaryotic cells, such as plant cells. Representative examples include: E. coli, Streptomyces, Agrobacterium; fungal cells such as yeast; plant cells, etc. In the present invention, host cells are preferably Agrobacterium.

To express polynucleotides of the present invention in higher eukaryotic cells, transcription can be enhanced, if enhancer sequences are inserted into vectors. Enhancers are DNA cis-acting factors, usually about 10 to 300 bp, acting on promoters to enhance gene transcription. One skilled in the art would know how to select suitable vectors, promoters, enhancers and host cells.

The obtained transformants can be cultured using conventional methods, expressing polypeptides encoded by genes of the present invention. Depending on host cells, culture media used for culturing may be selected from any conventional media. Culturing can be carried out under conditions suitable for host cell growth. When host cells grow to appropriate cell density, suitable methods (such as temperature shift or chemical induction) are used to induce selected promoters, and then culturing is continued for another period of time.

Recombinant polypeptides obtained from the above methods can be expressed in the cells, on cell membrane, or secreted out of the cells. If necessary, recombinant proteins can be isolated and purified using various isolation methods based on their physical, chemical, and other properties. These methods are well-known to one skilled in the art. Examples of these methods include, but are not limited to: conventional renaturation treatment, treatment with protein precipitating agents (salting out method), centrifugation, osmotic lysis of bacteria, ultrasonic treatment, ultracentrifugation, molecular sieve chromatography (gel filtration), adsorption chromatography, ion exchange chromatography, high performance liquid chromatography (HPLC) and various other liquid chromatography technology and a combination thereof.

Transforming plants may be achieved using Agrobacterium transformation or gene gun transformation, etc., such as Agrobacterium-mediated transformation of leaf disks. The transformed plant cells, tissues or organs can be regenerated into plants using conventional methods, resulting in plants with improved resistance to diseases.

### CIS-ACTING ELEMENTS

As used in this description, the term "cis-acting elements" refers to sequences that are located in the flanking regions of genes and can affect gene expression. Their functions are to participate in the regulation of gene expression. A cis-acting element itself does not encode any protein; it only provides an acting site. It interacts with trans-acting factors to result in its functions.

Through research, the inventors of the present invention discover: DST proteins of the present invention have DNA binding abilities. Their core binding elements are cis-acting elements, TGCTANN(A/T)TTG, wherein N represents A, C, G or T. The cis-acting elements of the present invention bind with DST transcription factors, increasing the sensitivity to drought and salt in plants, thereby lowering the drought and salt tolerance in plants. Said cis-acting elements preferably interact with a zinc finger domain of DST.

Conversely, if the interactions between the cis-acting elements and the DST transcription factors of the present invention are inhibited, it would result in enhanced drought and salt tolerance in plants.

### METHODS FOR ENHANCING DROUGHT AND SALT TOLERANCE IN PLANTS

As described in the present description, DST proteins of the present invention, their coding sequences or the bindings of DST proteins to cis-acting elements have a close relationship with drought and salt tolerance in plants. Inhibiting DST proteins, their coding sequences or the bindings of DST proteins to cis-acting elements would lead to enhanced drought and salt tolerance in plants.

Therefore, the present invention also provides methods for enhancing drought and salt tolerance in plants by inhibiting DST proteins, their coding sequences, or the bindings of DST proteins to cis-acting elements.

In one embodiment of the present invention, antagonists of DST proteins or their coding sequences can be used to inhibit their expression. Said antagonists include, but are not limited to: small molecule interfering RNA, antibodies, dominant negative regulators or antisense oligonucleotides. One ordinary skilled in the art, having known DST proteins or their coding sequences, would know how to use conventional methods and tests to screen and obtain said antagonists.

As used in the present invention, the term "non-conserved mutation" or "non-conservative mutation" refers to one or more amino acid or nucleotide substitution, deletion or insertion (preferably, non-conserved) in a DST protein or its coding sequence, resulting in enhanced drought and salt tolerance in plants.

In another embodiment of the present invention, methods known in the art may be used to introduce non-conserved mutations in the DST proteins of the present invention or their coding sequences. For example, nucleotide mutations in the DST protein coding sequences may be used to introduce non-conserved mutations in the amino acid sequences of DST proteins that contain SEQ ID NO: 2, endowing plants containing the mutant sequences with enhanced drought and salt tolerance. For example, mutation at amino acid 69, from asparagine to aspartic acid, or mutation at amino acid 162, from alanine to threonine.

In another embodiment of the present invention, transgenic plants with inhibited expression of DST genes or proteins can be prepared, and these transgenict plants may be optionally cross-bred with non-transgenic plants or other transgenic plants. For example, plants can be transformed with vectors containing small molecule interference RNA, antisense vectors, dominant negative regulation vectors specifically targeting DST proteins or their encoded proteins or host cells harboring said vectors.

### METHODS FOR SCREENING DROUGHT AND SALT TOLERANT PLANTS

According to the unique properties of the DST genes of the present invention and their encoded proteins, the present invention further includes methods for screening drought and salt tolerant plants.

In one embodiment, a screening method of the present invention include: (i) assessing, in a candidate plant, the level of a DST zinc finger protein transcription factor of the present invention, the expression level of its coding polynucleotide, and/or the level of binding of a cis-acting element of the present invention to a DST zinc finger protein transcription factor; (ii) comparing the level detected in the candidate plant in step (i) with the corresponding level in a control plant, if the level in the candidate plant is lower than that in the control plant, then the dandidate plant is a drought and salt tolerant plant.

In another embodiment, molecular marker selection techniques known in the art may be used to introduce drought and salt tolerant DST gene into other variants to screen for and culture new variants that are drought and salt tolerant. Said methods may use conventional cross-breeding methods. Its advantage is in that no gene transfer is required, avoiding safety concerns of gene transfer. Said methods may include: designing molecular markers specific for non-conserved mutant sequences, using said molecular markers to screen offsprings from cross-breeding of mutants having the non-conserved mutant sequences and other rice variants, thereby selecting individual plants harboring said non-conserved mutant sequences.

### MAIN ADVANTAGES OF THE PRESENT INVENTION

Main advantages of the present invention include:
(1) identifying DST genes and their encoded proteins or polypeptides, and confirming their relationship with drought and salt tolerance in plants, thereby providing new methods for studying drought and salt tolerance in plants;
(2) providing transgenic plants having enhanced salt or drought stress resistance, thereby providing excellent raw materials and products for producing and processing grains, cotton and oils; and
(3) providing methods for screening for drought and salt tolerant offsprings using molecular markers, which can be realized using conventional cross-breeding methods, without the need for gene transfer, thereby avoiding safety concerns of gene transfer.

The present invention provides new approaches to improving resistance to salt or drought stress in plants with great potential in applications.

### EXAMPLES

The following description, combined with specific examples, further illustrates the present invention. It should be understood that these examples are used to explain the present invention and should not be used to limit the scope of the present invention.

In the following examples, when conditions not specified in experimental methods, they are usually based on conventional conditions (for example, please see, Sambrook et al, "Molecular Cloning: A Laboratory Manual," third edition, 2001, Cold Spring Harbor Laboratory Press) or conditions according to manufacturers' suggestions. Unless otherwise indicated, percentages and ratios are calculated based on weights.

Unless otherwise defined, all professional and scientific terms used in the present description have the same meanings as those well known to one skilled in the art. In addition, any methods and materials similar or equivalent to those described in the present description may be used in the present invention. Preferred methods and materials described in the present description are used only for illustration.

Various media used in the examples (YEB liquid culture medium, AB liquid culture medium, AAM liquid culture medium, N6D2 culture medium, N6D2C culture medium, co-culture medium, selection culture medium N6D2S1, N6D2S2, pre-differentiation culture medium, differentiation culture medium, 1/2 MS0H culture medium, rice culture medium, SD culture medium, etc.) are prepared according to the descriptions in related literatures (Molecular Cloning: Laboratory Manual (New York: Cold Spring Harbor Laboratory Press, 1989; Hiei, Y., etc., Plant J., 1994, 6, 271-282).

### EXAMPLE 1: RICE DST GENE TRANSFER EXPERIMENTS

### 1. Generation of DST mutants having high drought and salt tolerance, its characteristics and subcellular localization

Rice seeds are treated with 0.6% of EMS (ethyl methanesulfonate) to construct a rice mutant library containing about 9,000 rice mutant lines. Large-scale screenings of rice mutant library were carried out under salt stress of 140mM sodium chloride. Salt- and drought-tolerant phenotypes were verified by subjecting candidate mutants to repeated salt stress of 140mM sodium chloride and 20% PEG4000 simulated drought stress. A highly drought- and salt-tolerant mutant (dst) is obtained.

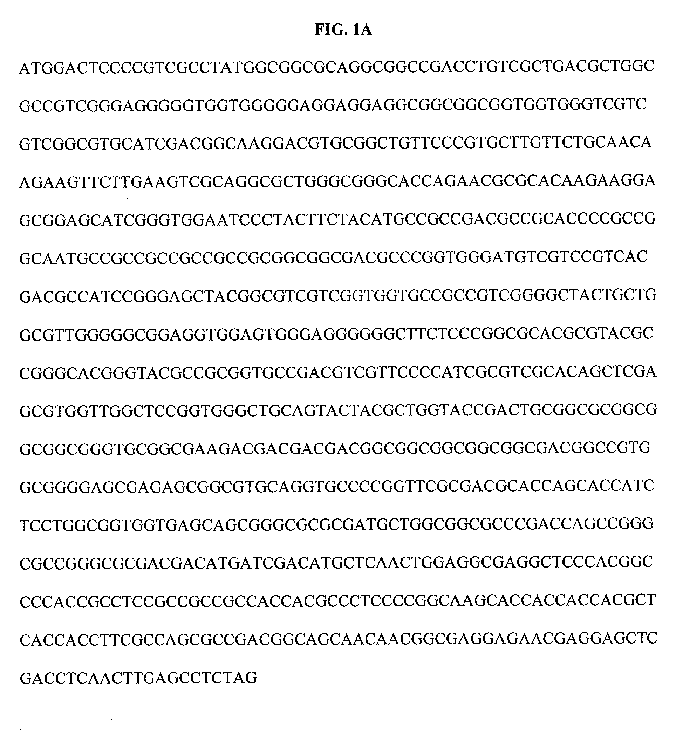

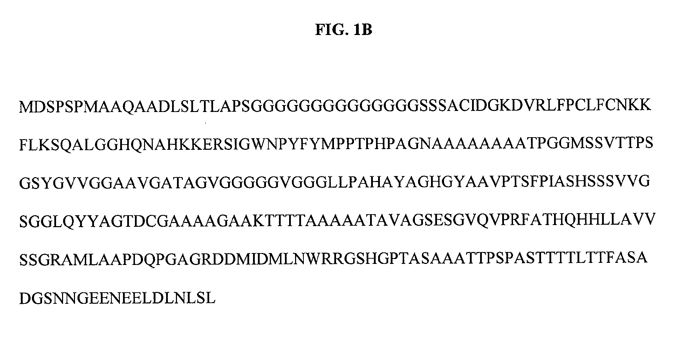

Using molecular markers, DST gene is preliminarily located on rice chromosome 3. By cross-breeding dst mutant with salt-sensitive strains, a large-scale F2 offsprings are constructed. Using molecular markers to screen for cross-bred offsprings from the group, combined with genotypes and phenotypes of the cross-bred offsprings, map-based cloning was performed. This led to successfully cloning of a DST gene. Said DST gene encodes a zinc finger protein (transcription factor) of unknown function having a conserved C2H2 type zinc finger domain. No other DST homologous copy is found in rice genome, and no homologous gene is found in Arabidopsis genome. The length of said genomic gene is 906 bp, without introns. The full-length ORF (open reading-frame) is 906 bp long, encoding 301 amino acids. The molecular weight of the protein product is estimated to be 29KDa (FIG. 1). Sequence comparison analysis shows that DST gene in this mutant contains 2 nucleotide mutations, which lead to 2 amino-acid substitutions (amino acid 69 is mutated from asparagine to aspartic acid, and amino acid 162 is mutated from alanine to threonine) and result in drought and salt resistant phenotype. This observation indicates that DST is a negative regulator for drought and salt resistance.

To determine subcellular localization of DST, a DST and GFP (green fluorescent protein) fusion construct is produced and transferred into onion epidermal cells using gene gun method for transient expression. The locations of fluorescence inside the cells are investigated using a fluorescence confocal microscope. Through this subcellular localization study DST is found to be specifically located in the nucleus.

### 2. Construction of transgene plasmids containing DST genomic fragments:

Wild-type rice BAC clones are digested with ApaLI restriction enzyme, followed by T4 DNA polymerase to generate blunt ends, which is then digested with SalI restriction enzyme. A 4.6-kb wild-type genomic fragment (containing full-length ORF of DST, promoter region, and stop codon with the downstream region) is thus recovered. A plant expression binary vector pCAMBIA1301 (purchased from CAMBIA) is digested with EcoRI, followed by T4 DNA polymerase to generate blunt ends, which is digested with SalI and then ligated with the recovered fragments mentioned above to successfully construct p-DST plasmid, which is used for transforming mutants and conducting complementation experiments. All enzymes are purchased from New England Biolabs.

### 3. Construction of DST-RNAi expression plasmids:

Use oligonucleotides at the 5 'and 3' ends as primers (SEQ ID NO: 4 and 5) to amplify DST fragment having the unique coding region (535-bp) by PCR. Ligate this fragment with the p1300RNAi vector (obtained by modifying pCAMBIA1300 through inserting a catalase intron as a linker, flanked by poly-A and poly-T at both ends) to construct DST-RNAi plasmid.

The 5 ' oligonucleotide primer sequence is:
5'-AAGCTTTCCTTGCGAAGCCAAATAGC-3' (SEQ ID NO. 4)

The 3 ' primer sequence is:
5'-GGATCCCGAGGCTCAAGTTGAGGTCGA-3' (SEQ ID NO. 5)

### 4. DST transgenic rice:

The two recombinant plasmids decribed above are transferred into Agrobacterium strain EHA105 using freeze-thaw method. Add 0.5-1 µg (about 10 µl) of plasmid DNA to each 200 µl of EHA105 competent cells, mix, and then place successively on ice, in liquid nitrogen and in a 37°C water bath for 5 minutes each. The reaction mixture is diluted to 1 ml with fresh YEB liquid culture medium and then incubated with shaking at 28°C for 2-4 hours. Take a 200 µl aliquot and spread it on a YEB plate containing kanamycin (Kan) antibiotics (50 µg/ml). Incubate the plate at 28°C for 2-3 days. Streak obtained colonies three times on YEB plates containing Kan (50 µg/ml) to select for single colonies.

Pick a single Agrobacterium colony from the YEB plate and inoculate it in 3 ml of YEB liquid culture media containing 50 µg/ml Kan antibiotics and incubate with shaking at 28°C overnight. On day 2, transfer 1% inoculum to 50 ml of AB liquid medium containing 50 µg/ml Kan antibiotics and continue incubation with shaking at 200 rpm until OD600 reaches about 0.6 to 0.8. Centrifuge fresh Agrobacterium culture at 5000 rpm and 4°C for 5 minutes. Collect and re-suspend the pellet in 1/3 volume of AAM liquid culture media. This suspension can be used to transform various rice recipient materials.

This experiment uses a conventional Agrobacterium-mediated transformation method to transform embryos callus of rice Zhonghua 11 (or its mutants). Immerse immature seeds of Zhonghua 11 (12-15 days after pollination) in 70% ethanol for 1 minute, sterilize them in a NaClO solution (mixed with water at 1:3, add 2-3 drops of Tween 20) for 90 minutes or more, and rinse the seeds with sterile water 4-5 times. Then, embryos from the seeds are picked out using a scalpel and a tweezer and plated onto N6D2 culture media to induce callus tissue formation, by culture at 26 ±1°C, in dark. After 4 days, they are ready for transformation.

Immerse the obtained embryo callus tissues in fresh AAM Agrobacterium liquid media with often shaking. Remove rice materials after 20 minutes, use sterile filter papers to remove excess bacteria solution, then transfer them onto N6D2C culture media covered with sterile filter papers, and coculture at 26°C for 3 days. Add Acetosyringone to co-culture media as the Agrobacterium Vir gene activator, at a concentration of 100 µmol/L.

After 3 days, remove callus tissues from the co-culture media, cut away germs and transfer them to N6D2S1 selection media (N6D2 medium containing 25 mg/l Hyg) for selection. After 7-12 days, transfer resistant callus tissues to N6D2S2 (N6D2 media containing 50mg/l Hyg) selection media and continue the selection.

After 10-12 days, transfer the vigorously growing resistant callus tissues to pre-differentiation culture media and incubate for about a week. Then, transfer them to differentiation culture media to allow them to differentiate (12 h light/day). Once the regenerated seedlings grow roots in 1/2 MSOH culture media, they are transfer to pot soil and grown in climate controlled chambers.

After the regenerated plants survive the transplantation, identify positive transgenic plants using methods known in the art, by detecting β-glucosidase (Beta-glucuronidase, GUS, see Jefferson et al. EMBO J. 6, 3901-3907, 1987) or by smearing on leaves with 0.1% herbicide. Extract total DNA from the leaves of the positive transgenic plants, and use PCR to further verify these transgenic plants.

In subsequent experiments, the T2 generation transgenic plants obtained by the above methods are used to investigate the drought and salt tolerant phenotypes, under drought and salt stress (140 mM NaCl) treatments, to confirm the functions of DST gene.

### EXAMPLE 2: TRANSGENIC PLANT CULTIVATING AND DROUGHT AND SALT STRESS TESTING

Take the seeds of transgenic rice obtained from EXAMPLE 1 and incubate them in an oven at 45°C for a week to break dormancy. Then, soak them in tap water at room temperature for 3 days, and prime them to germinate at 37°C for 2 days. After germination, spot seeding them in 96-well plates. Then, transfer them to light incubators, incubate them at 30°C, and expose them to light for 13 hours a day. After one day, gradually decrease the temperature to 28°C and 26°C and incubate them for one day each, and culture them at 20°C at night. After seedlings are all grown, replace the tap water with rice culture media and continue culturing.

After about 14 days of culturing, seedlings grow to a state of two leaves and one heart. Subject them to salt treatment in rice culture media containing 140 mM NaCl for 12 days, or PEG treatment in rice culture media containing 20% (m/v) PEG-4000 for 7 days to simulate draught stress.

With regard to the drought treatment in PVP pipes (polyvinylpyrrolidone pipe, 1.2 m high, 20 cm in diameter, two drain holes at the bottom of the pipe), transplant seedlings grown in water incubator for 25 days into the PVP pipes containing soil and culture the seedlings in a climate controlled chamber. The temperature is 24°C∼30°C, and the humidity is 50%∼60%. Drain water 30 days after transplantation, open the bottom drain holes to drain water, and perform drought treatments for 12 days.

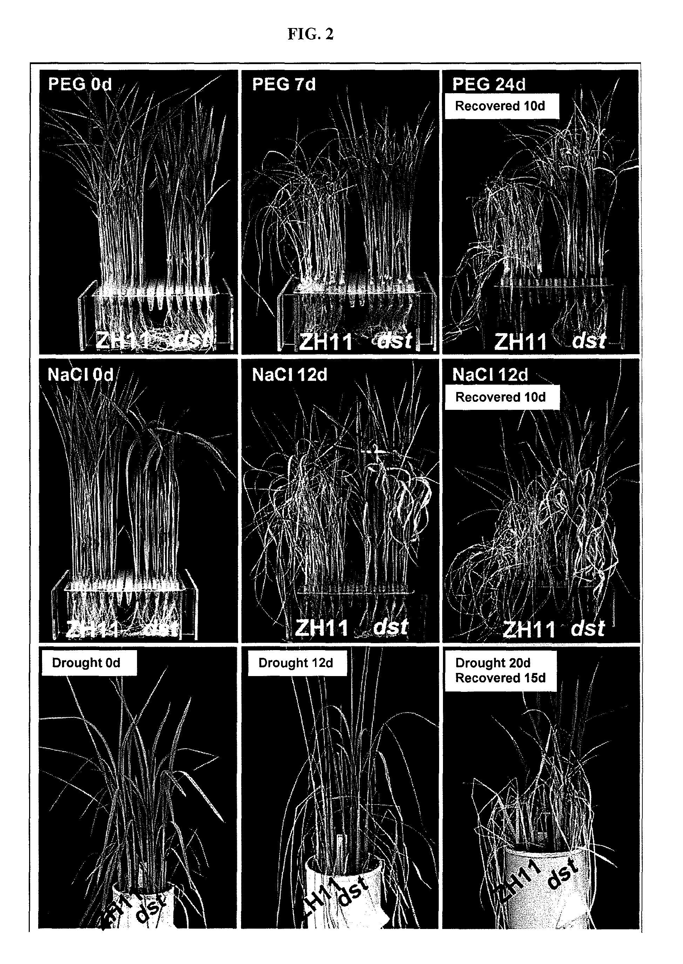

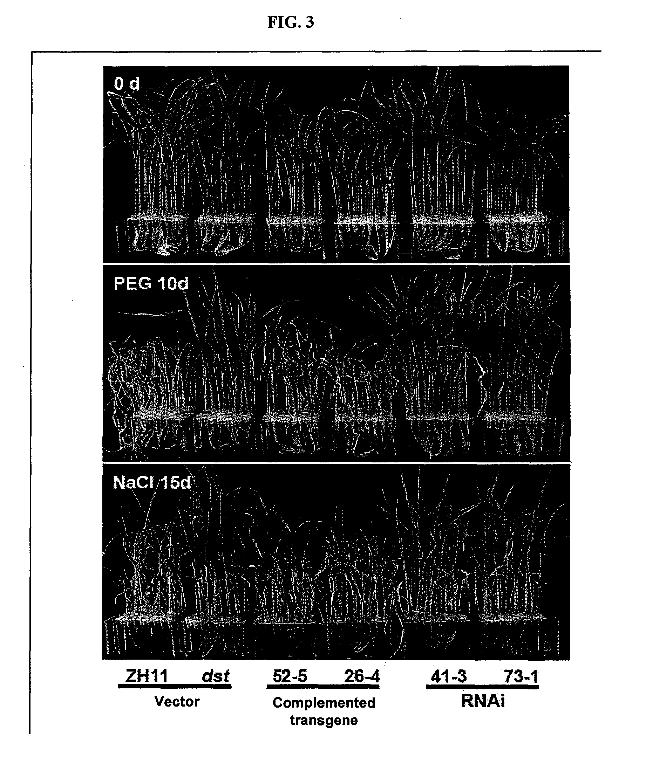

FIG. 2 and FIG. 3 show the experimental results. As shown in FIG. 2, rice mutant dst has significantly higher drought and salt tolerance than the wild type (Zhonghua 11, ZH11). Through observation and comparison, it is also found: as compared with the wild-type, mutants have more hydrogen peroxide (H2O2) accumulated around stomata, smaller stomatal aperture, relatively higher water contents in leaves under drought stress. Thus, mutants have higher drought resistance. In addition, because mutants have smaller stomatal aperture, lower stomatal conductance, and slower water vaporization rates, thereby reducing transportation of Na+ ions from roots to above ground parts (leaves, etc.) and lowering Na+ toxicity, and hence, increased salt tolerance.

As shown in FIG. 3: transfecting the DST genomic fragment from the wild-type rice (Zhonghua 11, ZH11) into a dst mutant restores the drought and salt sensitive phenotype of the wild-type in the transgenic complemented plants; whereas when the expression level of DST is reduced by RNAi (transforming Zhonghua 11), the drought and salt tolerance in Zhonghua 11 is significantly enhanced.

These results show: DST gene is successfully cloned and with genetically engineering involving DST, stress resistance in rice can be significantly increased.

### EXAMPLE 3: ANALYSIS OF DST TRANSCRIPTIONAL ACTIVATION ACTIVITY

Matchmaker GAL4 yeast two-hybrid system 3 (Clontech) is used to analyze the transcriptional activation of DST. To construct positive control vector pAD, NLS and GAL4 activating domain (AD) sequences are amplified by PCR and inserted into pGBKT7 (purchased from Clontech) BamHI/SalI cutting sites (primers are SEQ ID NO: 6 and 7) to fuse with the GAL4 DNA binding domain (BD) in pGBKT7.

Then, PCR is used to amplify DST full-length ORF (primers are SEQ ID NO: 8 and 9). After confirmation by sequencing, the PCR product is constructed into pGBKT7 vector at the BamHI and SalI sites to fuse with GAL4 DNA binding domain to obtian the pGBKT7-DST vector. Various vectors are then transformed into yeast AH109. After growing overnight, the culture is diluted and plated on SD culture media without Trp or without three amino acids (-Trp/-His/-Ade). Then, observe growth of the yeasts and determine the transcriptional activation activity of DST.

Oligonucleotide primer sequences are:
5'-AAAGGATCCAAGCGGAATTAATTCCCGAG-3' (SEQ ID NO: 6);
5'-AAAGTCGACCCTCTTTTTTTGGGTTTGGTGG-3' (SEQ ID NO: 7);
5'-AAAGGATCCTGATGGACTCCCCGTCGCCT-3' (SEQ ID NO: 8);
5'-AAAGTCGACCGAGGCTCAAGTTGAGGTCGAG-3' (SEQ ID NO: 9).

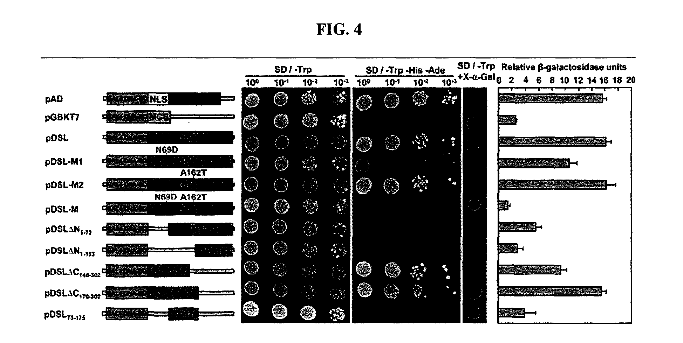

The results are shown in FIG. 4. As shown in the figure,: rice DST protein has transcriptional activation activity, whereas mutant DST proteins and proteins with N-terminal deletion lose transcriptional activation activity.

The results indicate that pGBKT7-DST has a stronger transcriptional activation activity, and the transcriptional activation domain is located at the N terminus, indicating that DST is a transcription factor with transcriptional activation activity.

### EXAMPLE 4: ANALYSIS OF DST PROTEINS AND ELECTROPHORESIS NOBILITY SHIFT ASSAY

### 1. Prokaryotic proteins expression:

Digest pGBKT7-DST vector with *EcoR*I and *Sal*I to obtain DST full-length cDNA, which is then reconstructed into pET32a(+). Transfect the prokaryotic expression vector pET32a(+) containing the recombinant DST into BL21, use IPTG to induce prokaryotic expression, and then purify the proteins using His-tag columns (beads).

### 2. Antibody generation:

Immunize rabbits with the purified proteins described above using conventional methods to generate anti-DST antibodies.

### 3. Synthesize probes, label them with biotin, purify them on PAGE gels, and recover the labeled probes by electroelution.

### 4. Allowed the labeled probes to react with the purified prokaryotic expressed DST proteins. Then, the complexes are subject to native-PAGE electrophoresis and transferred onto nylon membranes using semi-dry membrane transfer methods. The nylon membranes are then exposed to X-ray films for autoradiography to observe shift bands.

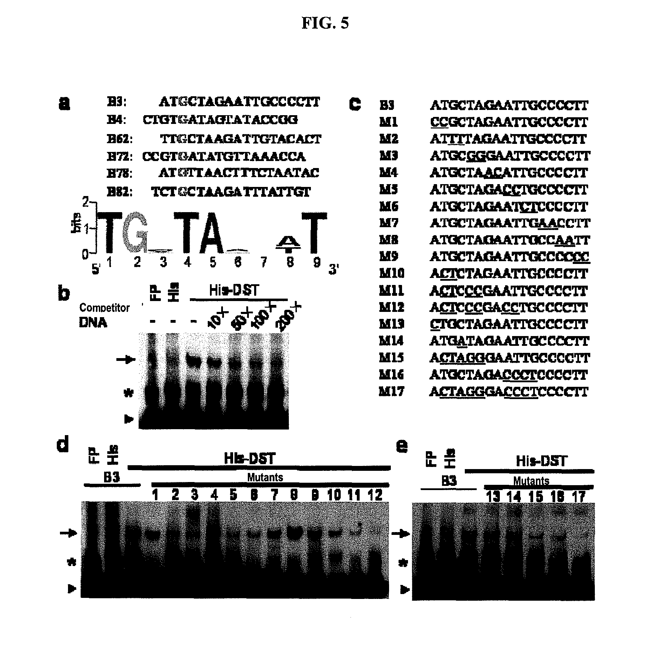

FIG. 5 shows the experimental results. As shown in FIG. 5, DST has DNA-binding ability. The core element for DST binding is: a *cis*-acting element TGCTANN(A/T)TTG (SEQ ID NO: 3).

The present study shows that DST binding to said *cis*-acting element can regulate the expression of downstream genes, thereby affecting the drought and salt tolerance in plants. Therefore, DST binding to the *cis*-acting element plays an important role in negative regulation of drought and salt tolerance.

### EXAMPLE 5: EXISTENCE OF DST GENE HOMOLOGS IN DIFFERENT PLANTS

Database search (http://plantta.jcvi.org/index.shtml) reveals, one DST gene homolog in sorghum (Sorghum bicolor) genome, with 54.3% protein similarity; three gene homologs in maize (Zea mays) genome, with 51.7%, 36.1%, and 33.5% protein similarities; one DST gene homolog in barley (Hordeum vulgare) genome, with 38.4% protein similarity; three gene homologs in sugarcane (Saccharum officinarum) genome, with 38.2%, 38.2% and 34.5% protein similarities.

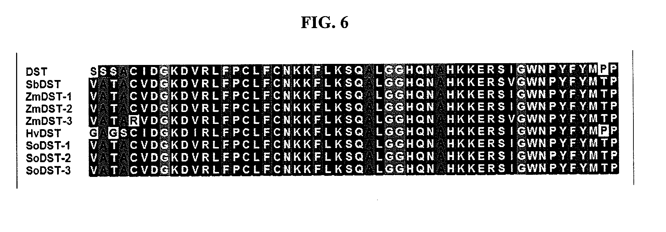

All these gene homologs have conserved C2H2-type zinc finger domains. They share identical zinc finger domain. At the same time, the similarities are high in the N-terminus domains (FIG. 6 shows the shared sequence of these gene homologs. The shared sequence is DGKDVRLFPCLFCNKKFLKSQALGGHQNAHKKERSIGWNPYFYM, i.e., positions 42-85 in SEQ ID NO: 2). C2H2 type zinc finger proteins bind the cis-acting elements via the zinc finger domains. Therefore, there is a corresponding relationship between these gene homologs and the cis-acting elements, indicating that DST gene homologs of other Gramineae crops may share similar functions as that of the rice DST gene.

### EXAMPLE 6: APPLICATIONS OF RICE AND OTHER CROPS DST GENE MOLECULAR MARKER-ASSISTED SELECTION TECHNIQUES IN CROP BREEDING TO IMPROVE RESISTANCE IN CROPS

Through chemical mutagenesis (EMS), two nucleotide mutations in the *DST* gene are produced (A is mutated to G at nucleotide 205, and G is mutated to A at nucleotide 484), causing two amino acid substitutions (asparagine is mutated to aspartic acid at position 69, and alanine is mutated to threonine at position 162), resulting in drought-resistant and salt-resistant phenotype. Design the following two primer pairs, SNP5 and SNP3, in said gene, so that amplified products include the first point mutation and the second point mutation, respectively.
SNP-5S: ATGGACTCCCCGTCGCCT (SEQ ID NO: 10)
SNP-5A: GTGCGCCGGGAGAAGCCC (SEQ ID NO: 11)
SNP-3S: GCGGTGCCGACGTCGTTCCC (SEQ ID NO: 12)
SNP-3A: GCCGCCGTCGTCGTCGTCTTC (SEQ ID NO: 13)

The first point mutation generates a ScrFI restriction enzyme cutting site, whereas the second point mutation destroys BstUI restriction enzyme cutting site. Digesting the amplified products obtained with primers SNP5 with ScrFI yields fragments of 311 bp, 85 bp, and 31 bp in the wild-type, whereas the mutant emplified product would yield fragments of 202 bp, 109 bp, 85 bp, and 31 bp, thereby producing polymorphism. Digesting the amplified products obtained with SNP3 primers with BstUI yields fragments of 66 bp, 40 bp, and 25 bp in the wild-type, whereas the mutant amplified product would yield fragments of 91 bp and 40 bp, thereby also producing polymorphism. Therefore, these two primer pairs SNP5 and SNP3 can be used as molecular markers, for use in molecular marker-assisted selective breeding.

Use the drought- and salt-resistant dst mutants to cross with rice variants, and screen the offsprings for one molecular marker or two molecular markers to select individuals carrying a DST mutant gene. Then, culture the new variants (lines) having increased drought- and salt-resistance.

Said method uses conventional cross-breeding methods, without gene transfer, thereby avoiding safety concerns associated with gene transfer. Therefore, such methods are advantageous.

All literatures cited in the present invention are used as references in the present application, as if every literature is singularly referenced. In addition, it should be understood, one skilled in the art having read the description of the present invention described above could change or modify various aspects of the present invention. These equivalents fall within the scope of the appended claims in the present application.

## Claims

1. A zinc finger protein transcription factor, **characterized in that** the transcription factor comprises: a polypeptide comprising the sequence of amino acids 42-85 of SEQ ID NO: 2, a conserved mutant polypeptide thereof, or a polypeptide homolog thereof.

2. The transcription factor of claim 1, **characterized in that** the polypeptide is selected from:
(a) a polypeptide comprising the amino acid sequence of SEQ ID NO: 2;
(b) a polypeptide derived from (a), having one or more amino acid residue substituted, deleted, or inserted, and capable of increasing sensitivity to drought and salt in plants; or
(c) a polypeptide homolog of the polypeptides of (a) - (b) comprising a Cys-2/His-2 type zinc finger structural domain and capable of increasing sensitivity to drought and salt in plants.

3. A polynucleotide, **characterized in that** the polynucleotide comprises a polynucleotide sequence coding for the polypeptide of claim 1.

4. The polynucleotide of claim 3, **characterized in that** a sequence of the polynucleotide is selected from:
(a) a sequence comprising the sequence of SEQ ID NO: 1;
(b) a sequence comprising the sequence of 1-435 of SEQ ID NO: 1; or
(c) a sequence complementary to one of the sequences of (a) - (b).

5. A vector, **characterized in that** the vector comprises the polynucleotide of claim 3.

6. A genetically engineered host cell, **characterized in that** the host cell comprises the vector of claim 5 or a genome having the polynucleotide of claim 3 integrated therein.

7. A cis-acting element, wherein the cis-acting element comprises the sequence of SEQ ID NO: 3 and can bind with the transcription factor of claim 1.

8. An inhibitor or a non-conserved mutant sequence of the zinc finger protein transcription factor of claim 1, or of the polynucleotide of claim 3.

9. A method for improving drought and salt tolerance in a plant, wherein the method comprises:
inhibiting the zinc finger protein transcription factor of claim 1,
inhibiting expression of the polynucleotide of claim 3, or
inhibiting binding between the cis-acting element of claim 7 and the zinc finger protein transcription factor of claim 1;
wherein, preferably, the method comprises using the inhibitor of claim 8 or producing the non-conserved mutant sequence of claim 8 in the plant, more preferably, introducing non-conserved mutations in the nucleotide sequence of SEQ ID NO: 1 or the amino acid sequence of SEQ ID NO: 2, or using the inhibitors of the nucleotide sequence or the amino acid sequence, more preferably, introducing a mutation at nucleotide 205 from A to G and a mutation at position 484 from G to A in the nucleotide sequence of SEQ ID NO: 1, or introducing a mutation at amino acid 69 from asparagine to aspartic acid and a mutation at amino acid 162 from alanine to threonine in the amino acid sequence of SEQ ID NO: 2.

10. A method to select for a drought- and salt-tolerant plant, wherein the method comprises:
(i) determining in a candidate plant a level of the zinc finger protein transcription factor of claim 1, a level of expresion of the polynucleotide of claim 3, and/or a level of binding between the cis-acting element of claim 7 and the zinc finger protein transcription factor of claim 1; and
(ii) comparing the level in the candidate plant determined in the step (i) with a corresponding level in a control plant, if the level in the candidate plant is lower than of the level in the control plant, then the candidate plant is a drought- and salt-tolerant plant.

11. Use of an inhibitor or a non-conserved mutant sequence of the zinc finger protein transcription factor of claim 1 or the nucleotide sequence of claim 3 in improving drought- and salt-tolerance in a plant;
wherein the inhibitor is preferably a small molecule interference RNA, an antibody, or an antisense oligonucleotide targeting the transcription factor or the nucleotide sequence.

12. The use of claim 11, **characterized in that** the improving drought- and salt-tolerance in the plant comprises:
(i) contacting the plant directly with the inhibitor;
(ii) introducing the non-conserved mutant sequence into the plant; or
(iii) designing a molecular marker specific for the non-conserved mutation sequence, using the molecular marker to select, from offsprings of hybridization between the mutant containing the non-conserved mutation sequence and a rice variant, an individual plant containing the non-conserved mutation sequence;
wherein the molecular marker comprises a primer pair of SEQ ID NO: 10 and SEQ ID NO: 11, and/or a primer pair of SEQ ID NO: 12 and SEQ ID NO: 13.

13. A method for improving drought- and salt-tolerance in a plant, wherein the method comprises:
(A) providing an inhibitor or a non-conserved mutant sequence of the zinc finger protein transcription factor of claim 1 or the nucleotide sequence of claim 3;
(B) subjecting the plant to one or more treatments selected from:
(i) contacting the plant directly with the inhibitor;
(ii) indroducing the non-conserved mutant sequence into the plant; or
(iii) designing a molecular marker specific for the non-conserved mutation sequence, using the molecular marker to select, from offsprings of hybridization between the mutant containing the non-conserved mutation sequence and a rice variant, an individual plant containing the non-conserved mutation sequence;
wherein the molecular marker comprises a primer pair of SEQ ID NO: 10 and SEQ ID NO: 11, and/or a primer pair of SEQ ID NO: 12 and SEQ ID NO: 13.

14. A method for producing a transgenic plant, **characterized in that** the method comprises:
(1) transforming a plant cell, a plant tissue, or a plant organ with a construct containing a non-conserved mutant sequence of the zinc finger protein transcription factor of claim 1 or a non-conserved mutant sequence of the polynucleotide of claim 3;
(2) selecting a plant cell, a plant tissue or a plant organ transformed by the non-consered mutant sequence; and
(3) regenerating a plant from the plant cell, the plant tissue or the plant organ from step (2),
wherein the regenerated plant has a higher drought- and salt-tolerance than a non-transformed plant.


Sequence Listing

<110>  LIN, Hongxuan
       HUANG, Xinyuan
       CHAO, Daiyin
       GAO, Jiping
       ZHU, Meizhen
       SHI, Min

<120>  Rice Zinc Finger Protein Transcription Factor DST and
       Use Thereof for Regulating Drought and Salt Tolerance

<130>  17257/017001

<150>  PCT/CN10/071587
<151>  2010-04-07

<150>  CN 200910048955.3
<151>  2009-04-08

<160>  13

<170>  PatentIn version 3.3

<210>  1
<211>  906
<212>  DNA
<213>  Oryza sativa


<220>
<221>  CDS
<222>  (1)..(906)

<400>  1
atg gac tcc ccg tcg cct atg gcg gcg cag gcg gcc gac ctg tcg ctg        48
Met Asp Ser Pro Ser Pro Met Ala Ala Gln Ala Ala Asp Leu Ser Leu
1               5                   10                  15

acg ctg gcg ccg tcg gga ggg ggt ggt ggg gga gga gga ggc ggc ggc        96
Thr Leu Ala Pro Ser Gly Gly Gly Gly Gly Gly Gly Gly Gly Gly Gly
            20                  25                  30

ggt ggt ggg tcg tcg tcg gcg tgc atc gac ggc aag gac gtg cgg ctg       144
Gly Gly Gly Ser Ser Ser Ala Cys Ile Asp Gly Lys Asp Val Arg Leu
        35                  40                  45

ttc ccg tgc ttg ttc tgc aac aag aag ttc ttg aag tcg cag gcg ctg       192
Phe Pro Cys Leu Phe Cys Asn Lys Lys Phe Leu Lys Ser Gln Ala Leu
    50                  55                  60

ggc ggg cac cag aac gcg cac aag aag gag cgg agc atc ggg tgg aat       240
Gly Gly His Gln Asn Ala His Lys Lys Glu Arg Ser Ile Gly Trp Asn
65                  70                  75                  80

ccc tac ttc tac atg ccg ccg acg ccg cac ccc gcc ggc aat gcc gcc       288
Pro Tyr Phe Tyr Met Pro Pro Thr Pro His Pro Ala Gly Asn Ala Ala
                85                  90                  95

gcc gcc gcc gcg gcg gcg acg ccc ggt ggg atg tcg tcc gtc acg acg       336
Ala Ala Ala Ala Ala Ala Thr Pro Gly Gly Met Ser Ser Val Thr Thr
            100                 105                 110

cca tcc ggg agc tac ggc gtc gtc ggt ggt gcc gcc gtc ggg gct act       384
Pro Ser Gly Ser Tyr Gly Val Val Gly Gly Ala Ala Val Gly Ala Thr
        115                 120                 125

gct ggc gtt ggg ggc gga ggt gga gtg gga ggg ggg ctt ctc ccg gcg       432
Ala Gly Val Gly Gly Gly Gly Gly Val Gly Gly Gly Leu Leu Pro Ala

130                 135                 140

cac gcg tac gcc ggg cac ggg tac gcc gcg gtg ccg acg tcg ttc ccc      480
His Ala Tyr Ala Gly His Gly Tyr Ala Ala Val Pro Thr Ser Phe Pro
145                 150                 155                 160

atc gcg tcg cac agc tcg agc gtg gtt ggc tcc ggt ggg ctg cag tac      528
Ile Ala Ser His Ser Ser Ser Val Val Gly Ser Gly Gly Leu Gln Tyr
                165                 170                 175

tac gct ggt acc gac tgc ggc gcg gcg gcg gcg ggt gcg gcg aag acg      576
Tyr Ala Gly Thr Asp Cys Gly Ala Ala Ala Ala Gly Ala Ala Lys Thr
            180                 185                 190

acg acg acg gcg gcg gcg gcg gcg acg gcc gtg gcg ggg agc gag agc      624
Thr Thr Thr Ala Ala Ala Ala Ala Thr Ala Val Ala Gly Ser Glu Ser
        195                 200                 205

ggc gtg cag gtg ccc cgg ttc gcg acg cac cag cac cat ctc ctg gcg      672
Gly Val Gln Val Pro Arg Phe Ala Thr His Gln His His Leu Leu Ala
    210                 215                 220

gtg gtg agc agc ggg cgc gcg atg ctg gcg gcg ccc gac cag ccg ggc      720
Val Val Ser Ser Gly Arg Ala Met Leu Ala Ala Pro Asp Gln Pro Gly
225                 230                 235                 240

gcc ggg cgc gac gac atg atc gac atg ctc aac tgg agg cga ggc tcc      768
Ala Gly Arg Asp Asp Met Ile Asp Met Leu Asn Trp Arg Arg Gly Ser
                245                 250                 255

cac ggc ccc acc gcc tcc gcc gcc gcc acc acg ccc tcc ccg gca agc      816
His Gly Pro Thr Ala Ser Ala Ala Ala Thr Thr Pro Ser Pro Ala Ser
            260                 265                 270

acc acc acc acg ctc acc acc ttc gcc agc gcc gac ggc agc aac aac      864
Thr Thr Thr Thr Leu Thr Thr Phe Ala Ser Ala Asp Gly Ser Asn Asn
        275                 280                 285

ggc gag gag aac gag gag ctc gac ctc aac ttg agc ctc tag              906
Gly Glu Glu Asn Glu Glu Leu Asp Leu Asn Leu Ser Leu
    290                 295                 300


<210>  2
<211>  301
<212>  PRT
<213>  Oryza sativa

<400>  2

Met Asp Ser Pro Ser Pro Met Ala Ala Gln Ala Ala Asp Leu Ser Leu
1               5                   10                  15


Thr Leu Ala Pro Ser Gly Gly Gly Gly Gly Gly Gly Gly Gly Gly Gly
            20                  25                  30


Gly Gly Gly Ser Ser Ser Ala Cys Ile Asp Gly Lys Asp Val Arg Leu
        35                  40                  45


Phe Pro Cys Leu Phe Cys Asn Lys Lys Phe Leu Lys Ser Gln Ala Leu
    50                  55                  60


Gly Gly His Gln Asn Ala His Lys Lys Glu Arg Ser Ile Gly Trp Asn
65                  70                  75                  80

Pro Tyr Phe Tyr Met Pro Pro Thr Pro His Pro Ala Gly Asn Ala Ala
                85                  90                  95

Ala Ala Ala Ala Ala Ala Thr Pro Gly Gly Met Ser Ser Val Thr Thr
            100                 105                 110

Pro Ser Gly Ser Tyr Gly Val Val Gly Gly Ala Ala Val Gly Ala Thr
        115                 120                 125

Ala Gly Val Gly Gly Gly Gly Gly Val Gly Gly Gly Leu Leu Pro Ala
    130                 135                 140

His Ala Tyr Ala Gly His Gly Tyr Ala Ala Val Pro Thr Ser Phe Pro
145                 150                 155                 160

Ile Ala Ser His Ser Ser Ser Val Val Gly Ser Gly Gly Leu Gln Tyr
                165                 170                 175

Tyr Ala Gly Thr Asp Cys Gly Ala Ala Ala Ala Gly Ala Ala Lys Thr
            180                 185                 190

Thr Thr Thr Ala Ala Ala Ala Ala Thr Ala Val Ala Gly Ser Glu Ser
        195                 200                 205

Gly Val Gln Val Pro Arg Phe Ala Thr His Gln His His Leu Leu Ala
    210                 215                 220

Val Val Ser Ser Gly Arg Ala Met Leu Ala Ala Pro Asp Gln Pro Gly
225                 230                 235                 240

Ala Gly Arg Asp Asp Met Ile Asp Met Leu Asn Trp Arg Arg Gly Ser
                245                 250                 255

His Gly Pro Thr Ala Ser Ala Ala Ala Thr Thr Pro Ser Pro Ala Ser
            260                 265                 270

Thr Thr Thr Thr Leu Thr Thr Phe Ala Ser Ala Asp Gly Ser Asn Asn
        275                 280                 285

Gly Glu Glu Asn Glu Glu Leu Asp Leu Asn Leu Ser Leu
    290                 295                 300

<210>  3
<211>  11
<212>  DNA
<213>  Artificial Sequence


<220>
<221>  misc_feature
<222>  (6)..(7)
<223>  any nucleotide

<220>

<221>  misc_feature
<222>  (8)..(8)
<223>  n=A or T

<400>  3
tgctannntt g                                                            11


<210>  4
<211>  26
<212>  DNA
<213>  Artificial Sequence

<220>
<223>  PCR Primer

<400>  4
aagctttcct tgcgaagcca aatagc                                            26


<210>  5
<211>  27
<212>  DNA
<213>  Artificial Sequence

<220>
<223>  PCR Primer

<400>  5
ggatcccgag gctcaagttg aggtcga                                           27


<210>  6
<211>  29
<212>  DNA
<213>  Artificial Sequence

<220>
<223>  PCR Primer

<400>  6
aaaggatcca agcggaatta attcccgag                                         29


<210>  7
<211>  31
<212>  DNA
<213>  Artificial Sequence

<220>
<223>  PCR Primer

<400>  7
aaagtcgacc ctcttttttt gggtttggtg g                                      31


<210>  8
<211>  29
<212>  DNA
<213>  Artificial Sequence

<220>
<223>  PCR Primer

<400>  8
aaaggatcct gatggactcc ccgtcgcct                                         29


<210>  9

<211>  31
<212>  DNA
<213>  Artificial Sequence

<220>
<223>  PCR Primer

<400>  9
aaagtcgacc gaggctcaag ttgaggtcga g                                    31


<210>  10
<211>  18
<212>  DNA
<213>  Artificial Sequence

<220>
<223>  PCR Primer

<400>  10
atggactccc cgtcgcct                                                   18


<210>  11
<211>  18
<212>  DNA
<213>  Artificial Sequence

<220>
<223>  PCR Primer

<400>  11
gtgcgccggg agaagccc                                                   18


<210>  12
<211>  20
<212>  DNA
<213>  Artificial Sequence

<220>
<223>  PCR Primer

<400>  12
gcggtgccga cgtcgttccc                                                 20


<210>  13
<211>  21
<212>  DNA
<213>  Artificial Sequence

<220>
<223>  PCR Primer

<400>  13
gccgccgtcg tcgtcgtctt c                                               21